(19) Europäisches Patentamt
European Patent Office
Office européen des brevets

(11) Veröffentlichungsnummer: **0 010 615**
**B1**

(12) # EUROPÄISCHE PATENTSCHRIFT

(45) Veröffentlichungstag der Patentschrift: **25.07.84**

(51) Int. Cl.³: **G 01 N 33/52,**
G 01 N 31/22

(21) Anmeldenummer: **79103651.0**

(22) Anmeldetag: **26.09.79**

(54) **Verfahren und Reagenz zur Bestimmung von Ionen, polaren und/oder lipophilen Substanzen in Flüssigkeiten.**

(30) Priorität: **02.10.78 DE 2842862**

(43) Veröffentlichungstag der Anmeldung:
**14.05.80 Patentblatt 80/10**

(45) Bekanntmachung des Hinweises auf die Patenterteilung:
**25.07.84 Patentblatt 84/30**

(84) Benannte Vertragsstaaten:
**AT BE CH DE FR GB IT LU NL SE**

(56) Entgegenhaltungen:
**DE - A - 2 724 486**
**GB - A - 1 339 667**

**ANGEWANDTE CHEMIE, Band 90, Heft 11, 1978, Weinheim J.P. DIX et al. "Ionenselektive Kronenether-Farbstoffe", Seiten 893 bis 894**

**CHEMISCHE BERICHTE, Band 109, Heft 5, 1976, Weinheim E. WEBER et al. "Neue Kronenäther und ihre Alkalimetallion-Komplexe", Seiten 1803 bis 1831**

(73) Patentinhaber: **BOEHRINGER MANNHEIM GMBH**
**Patentabteilung, Abt. E Sandhofer Strasse 112-132 Postfach 31 01 20**
**D-6800 Mannheim 31 Waldhof (DE)**

(72) Erfinder: **Vögtle, Friedrich, Prof. Dr.**
**Eisenachstrasse 25**
**D-5205 St. Augustin 2 (DE)**
Erfinder: **Dix, Johannes Peter**
**Rheinaustrasse 18**
**D-5300 Bonn-Beuel (DE)**
Erfinder: **Jaworek, Dieter, Dr.**
**Zöpfstrasse 4**
**D-8120 Weilheim (DE)**

(56) Entgegenhaltungen:
**CHEMIKER ZEITUNG, Band 97, Nr. 11, 1973 Heidelberg F. VÖGTLE et al. "Stereochemie im Innern cyclischer Verbindungen: Kronenäther, Katapinate und Kryptate", Seiten 600 bis 610**

**L.R. Sousa & J.M. Larson, Journ. Am. Chem. Soc. 99, 307-310 (1977)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 010 615 B1

Courier Press, Leamington Spa, England.

## Beschreibung

Die qualitative und/oder quantitative Bestimmung einzelner Ionen in physiologischen Flüssigkeiten ist für die Diagnose, Therapie und Verlaufskontrolle im medizinischen Bereich, aber auch in der Prozeßkontrolle chemischer und mikrobiologischer Verfahren von großer Bedeutung. In der klinischen Diagnostik sind bisher chemische Verfahren mit Farbstoffen, daneben spektralphotometrische Verfahren sowie Methoden mit ionen-selektiven Elektroden beschrieben worden.

Bei der Flammenemissionsanalyse (Flammenphotometrie) wird die Intensität des von angeregten Atomen emittierten Lichtes photoelektrisch bei der dem Element entsprechenden Wellenläge gemessen.

Im Gegensatz hierzu werden nach dem Verfahren der Atomabsorptionsspektroskopie nichtan geregte Atome benutzt, deren Konzentration um einige Zehnerpotenzen höher liegt. Die Vorteile dieser Methode sind die größere Empfindlichkeit sowie das Fehlen spektraler Interferenzen. Als Nachteile sind im Vergleich zur Emissionsanalyse aufzuzählen: der relativ große apparative Aufwand, sehr große Probevolumina (2 ml), geringe Probefrequenz sowie die getrennte und hintereinandergeschaltete Bestimmung beider Parameter. Obwohl dieses Verfahren zur Bestimmung von Natrium und Kalium als Referenzmethode angesehen wird, bleibt es dennoch auf medizinisch-wissenschaftliche Laboratorien beschränkt.

Zur einfachen und schnellen Bestimmung auch von Ionen-Konzentrationen bzw.—Aktivitäten in wäßrigen Lösungen sind ionen-selektive Elektroden (ISE) geeignet, die im Vergleich zu den spektralphotometrischen Verfahren einen geringeren apparativen Aufwand erforderlich machen. Nachteile dieser ionen-selektiven Elektroden sind die relativ lange und z.T. unterschiedliche Ansprechzeit der Elektroden, das Verstopfen des Elektroden-Sensors durch in der Probe vorhandene hoch- und niedermolekulare Substanzen, so daß diese über relativ geringe Stabilitäten verfügen.

Es war daher die Aufgabe der vorliegenden Erfindung, ein schnelles und zuverlässiges, sowohl im Flüssigtest als auch mittels Teststreifen durchführbares Verfahren zur Bestimmung von Ionen, polaren und/oder lipophilen Substanzen in Flüssigkeiten, insbesondere biologischen Flüssigkeiten, wie z.B. Blutserum, zu entwickeln.

Gelöst wird diese Aufgabe dadurch, daß man das zu bestimmende Ion oder die polare und/oder lipophile Substanz einwirken läßt auf einen selektiven Kimplexliganden bzw. ein selektives Wirtsmolekül aus der Gruppe der Cyclodextrane, Cyclophane, Kronenäther, Kryptanden, Podanden, Oligoester, Polyester, Oligoamide, Oligopeptide, Polypeptide, Oligoether und Polyether, welches durch eine direkte covalente Bindung mit einem Chromophor verknüpft ist und danach die Wellenlängenverschiebung im Absorptions-oder Emissionsspektruim oder die Extinktionsänderung naißt.

Als selektive Komplexiganden bzw. als selektive Wirtsmoleküle kommen cyclische oder acyclische medio- bzw. makromolekulare Verbindungen in Frage, welche gegenüber dem nachzuweisenden Ion oder der nachzuweisenden polaren und/oder lipophilen Substanz bereits als Komplexbildner bzw. als Wirtsmolekül vorliegen oder in Gegenwart derselben die zur Komplexbildung bzw. Adduktbildung in Form der Wirt-Gast-Wechselwirkung erforderliche Struktur annehmen. Die polaren Bereiche werden hierbei in Gegenwart des Ions zu diesem hin ausgerichtet. Ringgröße und Struktur des Wirtsmoleküls ergeben in Abhängigkeit vom wirksamen Durchmesser bzw. dem spezifischen polaren oder hydrophoben Charakter des komplexierten Gast-Ions bzw. Gast-Moleküls die Selektivität. Bei der Komplexbildung ändert der Ligand häufig seine Konformation, in der Regel wird sie fixiert, wodurch Komplexbildung und Komplexdissoziation beeinflußt werden. So eignen sich als Komplexliganden bzw. Wirtsmolekül Verbindungen vom Oligoether, Polyether, Oligoester-, Polyester, Oligoamid- und Polyamid-Typ. Gut geeignet sind z.B. Kronenether, Cryptanden, Podanden oder Derivate davon, sowie Cyclodextrine, cyclische Peptide oder Peptide, welche in Gegenwart des zu bestimmenden Ions oder der polaren Substanz die zur Komplexbildung erforderliche Sekundär, Tertiär- oder Quartär-Struktur annehmen. Weiterhin eignen sich Tetrahydrofuran-haltige Ester-verknüpfte Makrolide und analoge Verbindungen, die in bioligischen Systemen den Transport regulieren bzw. regulieren können. Auch kommen reine Kohlenwasserstoff-Gerüste wie Cyclophane liphophile Wirtsmoleküle ("lipophile Hohlräume") in Frage. Es sind auch Kombinationen obiger Funktionen möglich.

Die Derivate der Komplexiganden bzw. Wirtsmoleküle können Brücken oder Ketten aufweisen, welche Oligo- bzw. Polyethylenglykol-Gruppierungen oder andere Heteroatom-haltige Gruppierungen enthalten können.

Der kovalent, heteropolar oder hydrophob gebundene Chromophor ist ein Farbstoff oder Fluoreszenzfarbstoff oder ein Chromogen, dessen Absorptionsspektrum sich durch Wechselwirkung, wie ladungsverschiebung und Störung der Mesomerie entweder im Grund- oder/und angeregten Zustand durch die Gastteilchen, wie polare Ionen bzw. lipophile Gastmoleküle ändert. Als Farbstoffe kommen z.B. diejenigen des Polyen-, Merichinoid-, Chinon-, Azo- (wie Methylorange und Methylrot), Pyrrol-, Merocyanin-, Indigo-, Indophenol-, Stilben-, Azomethin-, Anthrachinon-, Naphtochinon-, Cyanin-, Phthalein-, Polymethin-, Alizarin-Typs in Frage.

Der im Einschlußkomplex bzw. im Wirtsmolekül enthaltene Chromophor kann ein saurer Farbstoff oder ein Salz davon sein, z.B. das Lithium-, Natrium-, Kalium-, Ammonium-, Calcium-, Alkylammonium- oder Magnesium-Salz. Der Farbstoff enthält hierbei bevorzugt eine Carboxylat-, Sulfonat-, Phenolat- oder Thiophenolat-Gruppierung. Die nachzuweisenden Ionen können Kationen und Anionen

sein, vor allem Alkalimetall-, also Lithium-, Natrium-, Kalium-, Ionen, Ammonium-Ionen, Erdalkalimetall-, also Magnesium- und Calcium-Ionen oder andere Metall-Ionen, wie Schwermetallionen wie Eisen-, Zink-, Kupfer-, Kobalt-, Nickel-, Molybdän- und Chrom-Ionen sein. Es sind vor allem auch organische Kationen, Beispielsweise Oligoalkylammonium- Ionen, Phosphonium-Ionen, Guanidin-Ionen, Cholin-Ionen nachweisbar. Als nachzuweisende Anionen sind insbesondere Chlorid-, Bromid-, Jodid-, Sulfat-, Nitrat-, Nitrit-, Phosphat-, Diphosphat-, Triphosphat-, Hydrogenphosphat-, Hydrogencarbonat-Ionen, zu nennen. Als neutrale polare Substanzen kommen neutrale Gastteilchen wie beispielsweise Harnstoff, Thioharnstoff, Guanin, Guanidin, Harnsäure, Cholin, Creatinin, eine Aminosäure, Zucker, als typische lipophile Gastmoleküle Steroide wie Cholesterin und Lipide wie Triglyceride, Lecithine, in Frage.

Die Kation- und Anionkonzentration sowie Konzentrationen von neutralen Gastteilchen lassen sich auf diese einfache Weise durch Farbeffekte, also photometrisch qualitativ und quantitativ erkennen.

Die Selektivität kann sich sowohl auf ein einziges, ganz bestimmtes nachzuweisendes Ion bzw. nachzuweisende Substanz als auch auf eine Ionengruppe bzw. Substanzgruppe beziehen. Hierzu sind z.B. Chromophore, wie Farbstoffe mit mehreren gleichen oder verschiedenartigen Komplexliganden oder Wirtsmolekülen, wie Kronenethern verschiedener Hohlraumgrößen einsetzbar, wie in Formel I dargestellt,

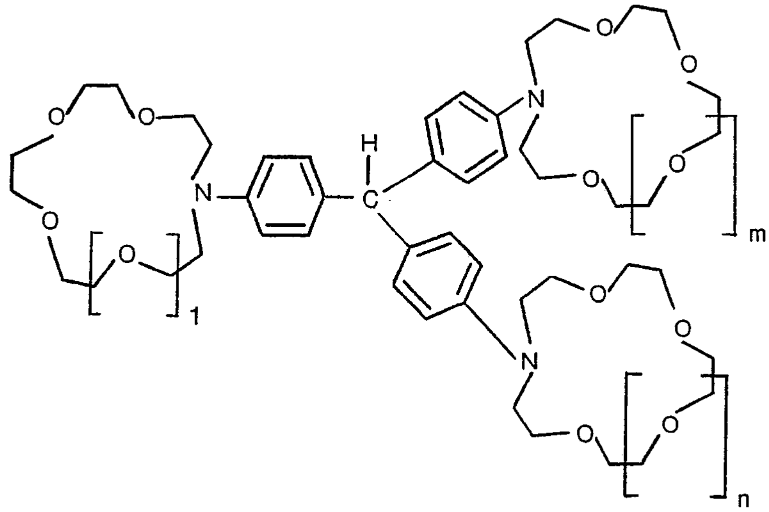

I

wobei 1, m und n bevorzugt die Zahlen 0 bis 4 bedeuten.

Weiterhin können an einen Komplexliganden oder ein Wirtsmolekül, wie das Kronenethergerüst ein oder mehrere, gleiche oder verschiedenartige Chromophore, wie Farbstoffe, angehängt werden. Der Farbumschlag kann beispielsweise auf folgendem Mechanismus gemäß Formel II beruhen

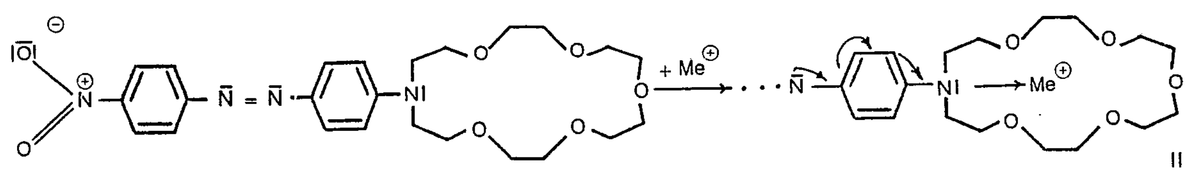

II

wobei Me$^{\oplus}$ beispielsweise ein Alkali-, Erdalkali-, Ammonium- oder Schwermetall-Ion bedeuten können und wobei das Mesomeriesystem gemäß Formel III zugrunde liegt, deren Gewicht und damit Energieinhalt im Grund- und angeregten Zustand in der Regel verschieden sind:

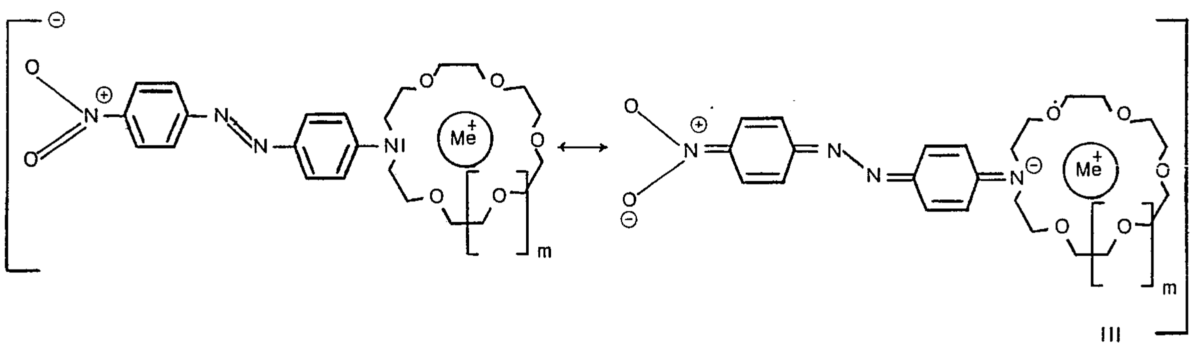

III

wobei m die Zahlen 0 bis 4 bedeuten.

Die Kronenether-, Cryptand- und Podand-Chemie beinhaltet beispielsweise eine Vielzahl von Methoden zur Knüpfung größerer und mittlerer Ringe sowie bi- und tricyclischer Systeme mit $-(CH_2)_n-$, aryl- und Heteroatom-haltigen Strukturelementen. Kronenether, Cryptanden und Podanden-Moleküle zeigen die Fähigkeit der Ausbildung stöchiometrischer kristalliner Komplexe sowie der selektiven bzw. spezifischen Komplexierung von beispielsweise Alkali-, Erdalkali-, Ammonium- und Schwermetallionen sowie von Neutralmolekülen. Über das Phasentransferverhalten der genannten Verbindungen liegen zahlreiche experimentelle Ergebnisse vor.

Zur Definition der Kronenether, Cryptanden und Podanden siehe die Übersichtsarbeiten F. Vögtle et al in Kontakte (E. Merck) 1/77, Seite 11; 2/77, Seite 16; 3/77, Seite 36; 2/78, Seite 16; 3/78, Seite 32; 1/79, Seite 3.

Trotz ausgedehnter Untersuchungen einerseits in der Kronenether-, Cryptand- und Podand-Chemie bezüglich Synthesen und Komplexierung als auch andererseits über die Ionenbeeinflussung der Farbe etwa bei Beizenfarbstoffen, Komplexometrie-Indikatoren und Porphyrin-Farbstoffen ist bisher über die Kombination dieser beiden Gebiete nichts bekannt geworden.

In L. R. Sousa, Journ.Am.Chem.Soc. *99*, 307—310 (1977) wird der Einfluß von Metall-Ionen auf die Fluoreszenzund Phosphoreszenz-Quanten-Ausbeute von an Kronenether gebundenem Naphthalin beschrieben. In Anmerkung (10) zu dieser Literaturstelle wird ausdrücklich darauf hingewiesen, daß Farbverschiebungen durch die Zugabe der Metall-Ionen praktisch nicht auftreten oder im Rahmen der Meßgenauigkeit der Spektrometer liegen.

Aus DE—A—21 23 256 sind makrocyclische Imine mit insgesamt 14 bis 60 Ringatomen bekannt, die als Komplexliganden mit einem Chromophor verknüpft sind. Die dort beschriebenen Komplexliganden eignen sich dazu, komplexgebundene Metallverbindungen in Medien, in denen sie normalerweise unlöslich sind, löslich zu machen sowie zur Abtrennung von Salzen. Ein Hinweise auf die Farbänderung durch Komplexierung läßt sich aus dieser Veröffentlichung nicht entnehmen.

Es gibt ein Reihe von Arbeiten über relevante Überbrückungen bei Cyclophanen und Kronenethern unter Ausnützung von Verdünnungsprinzip und Templateffekt. Es liegen umfassende Kenntnisse über Synthese und Komplexierungsverhalten von cyclischen Kronenethern und Cryptanden vor. Gleichfalls wurden im einzelnen Reaktionen an Kronenether-Systemen mit Erfolg durchgeführt. Die bischerigen Erfahrungen auf diesem Gebiet ermöglichen zum einen die Synthese geeigneter Kronenether- und Cryptandbausteine, zum anderen die Einführung von Oligoethylenglycoloder anderer Heteroatomhaltiger Brücken.

Es wurden eine Reihe von Azo- sowie Di- und Triphenylmethan-Farbstoffen aus dem Baustein gemäß Formel IV ($R_1$ = Wasserstoff, n = 1—3) und IV' hergestellt. An diesen Azofarbstoffen kann in Abhängigkeit von der Ringgröße eine selektive, für jedes Ion spezifische Verschiebung des sichtbaren Absorptionsmaximums in bestimmter Richtung beobachtet werden, die häufig mit einer Änderung der molaren Extinktion verbunden ist. Die größten der spektralen Veränderungen wie $\lambda$-max verursacht in der Regel das in einem bestimmten Kronenetherring innerhalb einer Gruppe des Periodensystems bezüglich des Radius optimal passende Kation.

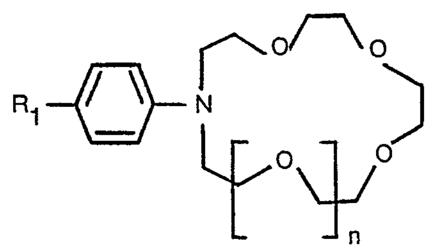

IV

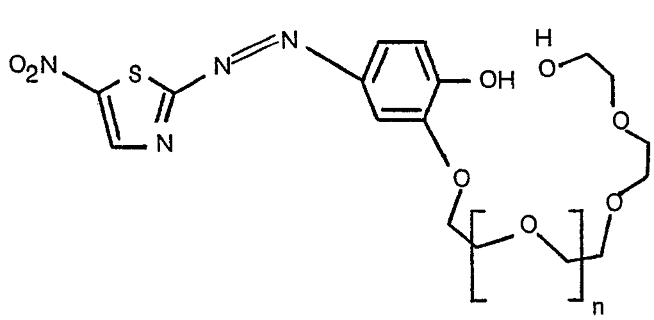

IV'

Es wurden verschiedene Farbstoffsysteme mit Kronenether- und Cryptand-Einheiten veränderlicher Ringgröße und Struktur versehen Hierbei beinhaltet ist auch die Variation in der Lipophilie bzw.

Hydrophilie innerhalb des Hohlraums und an der Peripherie des Kronenetherrings. Beide Strukturelemente sollen so miteinander verbunden sein, daß ein oder mehrere Donor-Heteroatome des Liganden gleichzeitig essentieller Bestandteil des Chromophors sind. Ein komplexiertes Kation greift an einer empfindlichen Stelle des Chromophors mehr oder weniger intensiv an und beeinflußt so seine Absorption spezifisch. Diese Einwirkung auf das absorbierende System hängt von Faktoren wie Größe des Ions, seiner Ladung bzw. Ladungsdichte, Solvatation sowohl des Farbstoffs wie auch des Kations bzw. Anions sowie dem Lösungsmittel ab.

Die Phenylaza-Kronenether des Typs der Formel IV eignen sich besonders wegen ihrer Analogie zu dem in der Farbstoffchemie viel verwendeten N,N-Dimethylanilin und ähnlichen Anilinen. Durch Azokupplung wurden Farbstoffe des Typs der Formel IV hergestellt, deren unverkronte Analoga als lichtechte Textilfarben bekannt sind, wobei beispielsweise n = 1 und $R_1$ bedeutet:

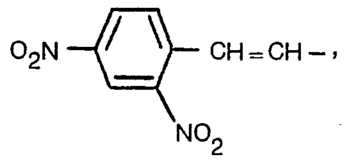

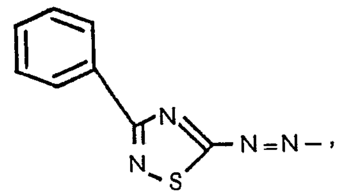

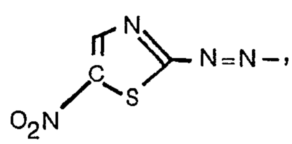

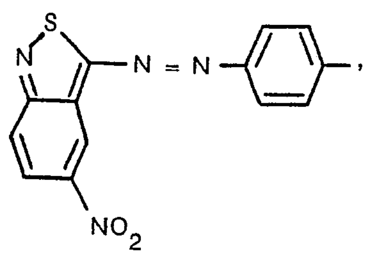

Der auxochrome Kronenetherstickstoff läßt hier eine hypsochrome Verschiebung des Absorptionsmaximums bei der Wechselwirkung mit dem Gastion zu, da sein freies Elektronenpaar nach erfolgter Komplexierung zur Mesomerie nicht mehr oder nur noch zum Teil, abhängig von der Art des komplexierten Ions (z.B. Ladung), zur Verfügung steht. Eine weitere Erhöhung der Ionenselektivität läßt sich durch teilweise Versteifung des Kronenetherrings erzielen, wie z.B. in dem Phenylaza-benzokronenether des Types V (wobei n = 1 bis 3 ist), die sich z.B. als Azokupplungspartner und für ähnliche Umsetzungen eignen.

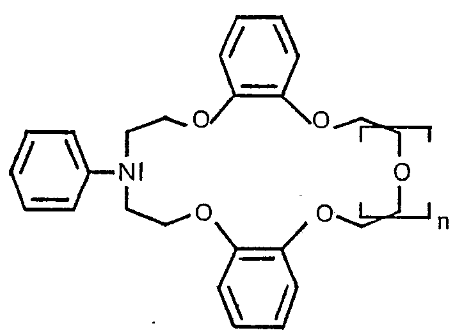

V

Hier kommen vor allem chromophore Systeme in Frage, bei denen eine leichte Beeinflußbarkeit durch ein komplexiertes Ion zu erwarten ist, beispielsweise solche mit hoher Solvatochromie, wie Indophenole des Typs IV, wobei $R_1$ bedeutet

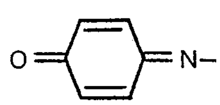 und 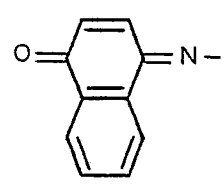

die sich auf dem Wege über Verbindungen der Formel IV, worin $R_1 = NO_2$ bzw. $R_1 = NH_2$ herstellen

lassen. Der letzte Schritte beinhaltet eine oxidative Kupplung mit Phenolen. Die analogen CH-Verbindungen gemäß Formel IV, wobei $R_1$ bedeutet

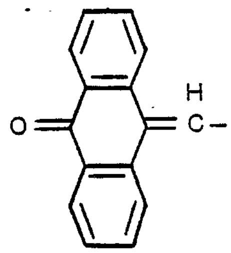

lassen sich aus den entsprechenden Aldehyden ($R_1 = CHO$) darstellen, die z.B. über eine Vilsmeier-Reaktion erhältlich sind.

Des weiteren eignen sich Stilbene und Merocyanine und evtl. Azaanaloge der Formel IV, wobei n = beispielsweise 1 und $R_1$ beispielsweise bedeutet:

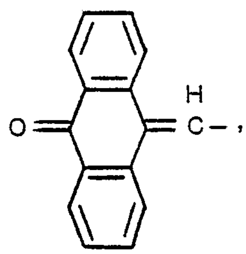

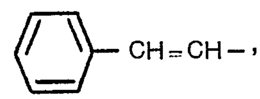

bzw.

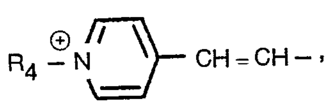

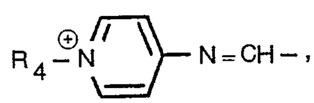

bzw.

und $R_4$ für Alkyl mit etwa 1—5 Kohlenstoffatomen, vorzugsweise 1 bis 2 Kohlenstoffatomen steht. Die Verknüpfung von Farbstoffen im obigen Sinne mit Cryptandsystemen führt zur Erreichung höherer Komplexstabilität, vor allen in wäßrigen Medium, und zu höherer Selektivität. Hier ist von allem die Kupplung reaktiver Diazoniumsalze mit Benzocryptanden der Formel VI

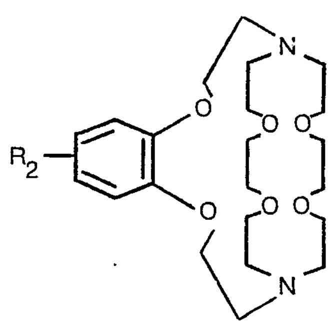

VI

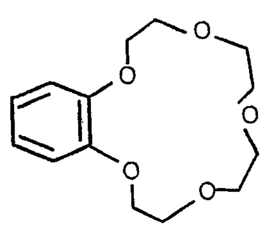

VII

vorteilhaft, wobei $R_2$ beispielsweise bedeutet

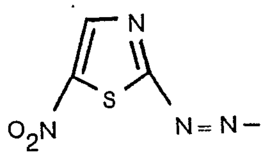

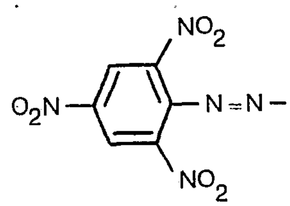

Als Modellsubstanzen für die Synthese haben sich Benzokronenether der Formel VII erwiesen, die gleichfalls über das zu erwartende Verhalten gegenüber Ionen Aufschluß geben. Die Variation der Hohlraumgröße verlangt Benzocryptanden, wie sie für Verbindungen der Formel VI benötigt werden, die jedoch um eine oder mehrere Oxyethyleneinheiten verkürzt oder verlängert sind.

Darüber hinaus sind Cryptanden des Typs der Formel VIII geeignet:

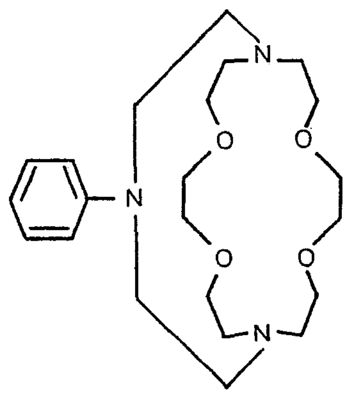

VIII

Auf ähnliche Weise sind auch solche des Typs der Formel IX herstellbar, die sich ähnlich wie die einfachen Phenylaza-Kronenether durch Reaktion in 4-Position des Anilingerüstes zu Farbstoffcryptanden umsetzen lassen, wie z.B. IXa und IXb, worin $R_3$ = eine Nitro-, Cyano-, Sulfonium- oder ähnliche Gruppe ist.

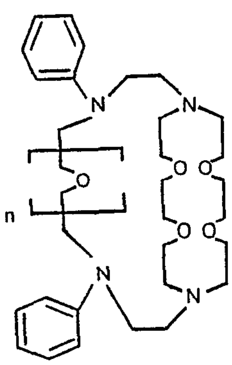

IX

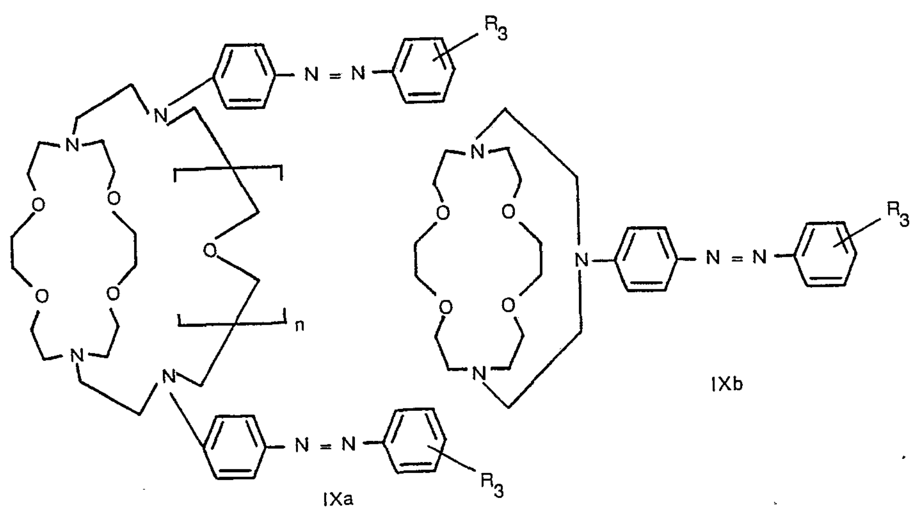

IXa

IXb

Neben den bischer genannten Typen, bei denen eine Mesomerie-Beeinflussung, z.B. durch Störung durch eingelagerte Ionen an einem Heteroatom stattfindet, kann auch ein Angriff am Antiauxochrom bzw. an einer Azobrücke stattfinden gemäß X

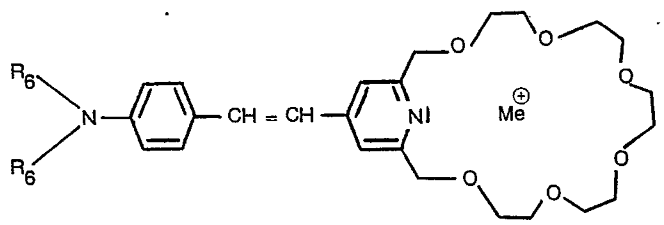 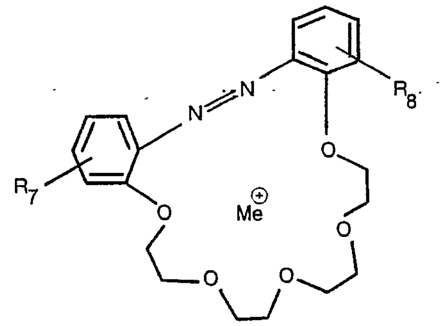

X

worin $R_6$ eine gegebenenfalls auch cyclische niedere Alkylgruppe, $R_7$ und $R_8$ gleich oder verschieden sind und Wasserstoff eine Nitro-, Cyano- oder Dialkylaminogruppe darstellen.

Beispiele für Fluoreszenzfarbstoff-Kronenether:

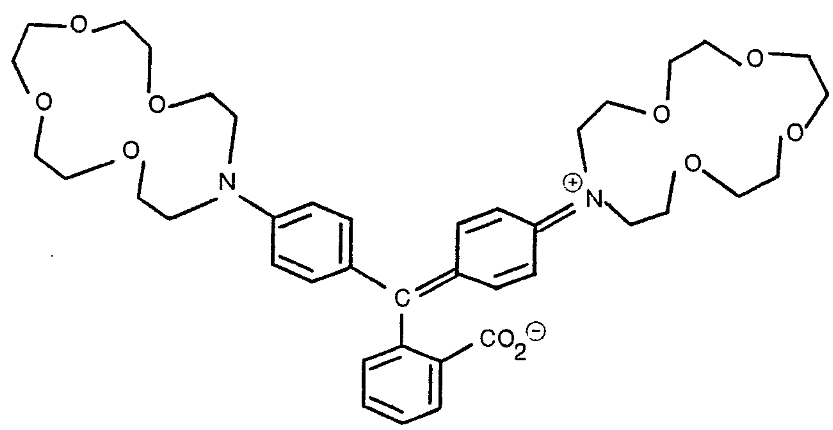

XIa

Weitere Beispiele für Farbstoff-Kronenether sind XIa (Fluoreszenzfarbstoffkronenether), XIb (worin $X = N$, CH; $Y = CH_2$, NH, O, S; $n = 1$ bis 3) wie Methylenblau, Kronenether XIc und XId (Azomethinfarbstoff-Kronenether, worin $R_9$ = niedriges Alkyl bedeutet), worin $A^{\ominus}$ ein übliches Anion darstellt,

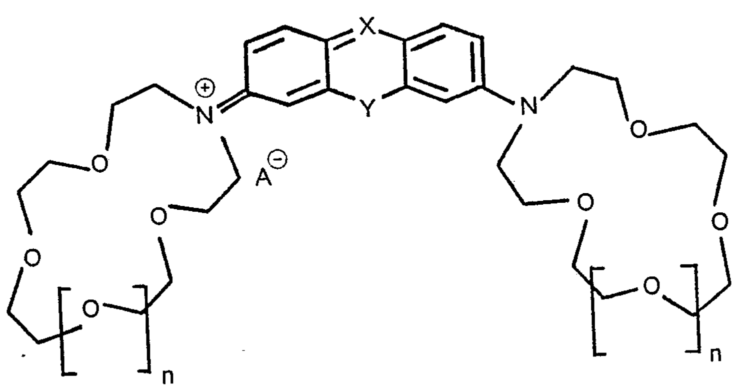

XIb

XIe (Anthracilin-Kronenether), XIf (Alizarin-Kronenether), XIg (Cyanin-Typ). Ein Beispiel für einen lipophilen Hohlraum ist die Verbindung der Formel XII, worin $n = 1$ bis 6 und $R_{10}$ Wasserstoff, eine niedere Alkyl- Oder Arylgruppe darstellt.

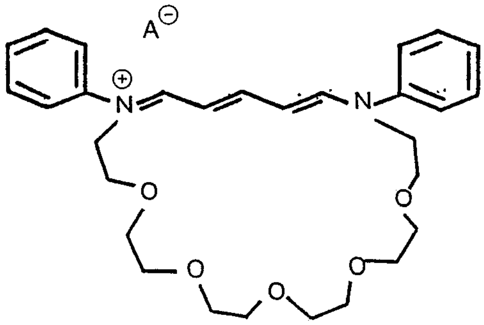

XIc

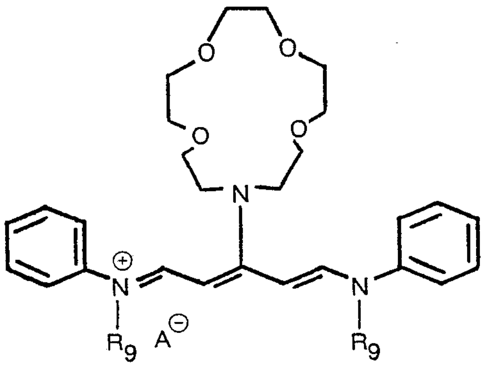

XId

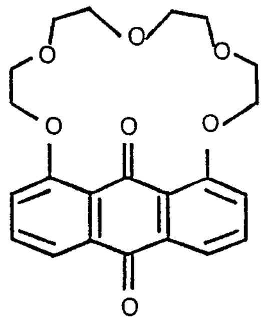

XIe

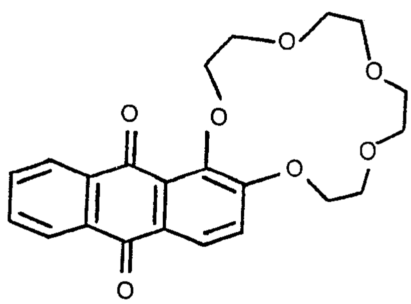

XIf

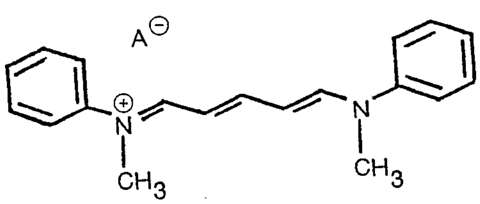

XIg

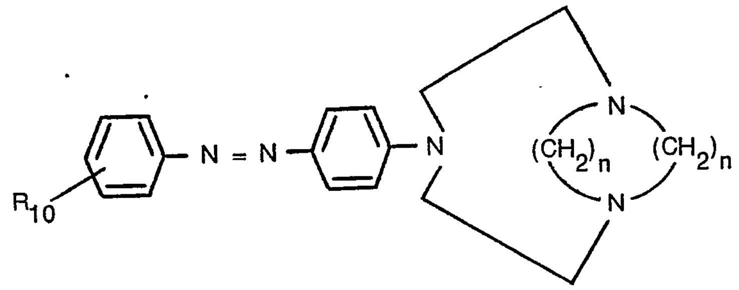

XII

Als Fluoreszenzfarbstoffe kommen insbesondere diejenigen aus der Gruppe der Phthaleine in Frage (Formel XIa), wobei Kronenetherringe an den Chromophor angebracht sind an den Stellen, die bei der Komplexierung besonders empfindlich beeinflußt werden. Bei solchen Fluoreszenzfarbstoffen liegt eine stärkere Störung der Farbe bzw. der Fluoreszenz (Phosphoreszenz) des Chromophors durch Komplexierung vor. Durch Einstellen des Kronenether- hohlraumes auf bestimmte Ionen können solche Wechselwirkungen und Effekte auf bestimmte Ionensorten selektiv eingestellt werden. Hier spielen außer den Alkali- und Erdalkalimetallionen die Schwermetallionen sowie Ammoniumionen und andere organische Oniumionen, wie Phosphoniumionen, eine Rolle. Es kommt hiermit ein Nachweise von Ammoniumgruppen-enthaltenden Desinfektionsmitteln in Frage.

Durch Einbringen von Farbstoffkronenethern (oder umgekehrt von Ionen) in Fasern bzw. Kunststoffolien usw. lassen sich durch einen Färbevorgang, der dem Beizen entsprechen kann, sowohl Farben auf Textilien usw. fixieren als auch modifizierte Färbevorgänge und Farbnuancen erzielen. Die Bindung ist dabei nicht ionogen und nicht kovalent, sondern geschieht über eine Kronenether-Wirt-Gast-Wechselwirkung, d. h. Ion-Dipol-Wechselwirkung; sie kann reversibel gestaltet werden. und somit analytisch ausgewertet werden.

Es eignen sich vor allem auch Anthrachinone mit funktionellen Gruppen, etwa Sulfonsäuregruppen, bzw. Naphthochinosulfonsäuren, die in der Färberei früher oder heute noch eingesetzt werden. Sie können erfindungsgemäß ionenselektiv modifiziert werden und so zum Nachweis bestimmter Ionen dienen, auch dadurch, daß sie auf Papier oder Fasern sowie Polymere aufgetragen bzw. gebunden sind.

Bei allen Farbstoffen, vor allem auch den Cyanin-Farbstoffen, sei auf die entsprechenden organisch-chemischen Lehrbücher bzw. Farbstoffbücher verwiesen. Ein sehr geeigneter Cyanin-Farbstoff ist das König'sche Salz (Formel XIg), welches einfach aus Pyridin und N-Methylanilin herstellbar ist.

Das König'sche Salz kann durch Anheften von Kronenethern so modifiziert werden, daß bei der Komplexbildung mit Alkali-bzw. Erdalkali- oder Schwermetallionen Farbänderungen bzw. Farbeffekte eintreten, die zum Nachweis bzw. zu Konzentrationsbestimmungen dieser Ionen, auch bei gleichzeitigem Vorliegen anderer Ionen, eingesetzt werden können.

Eine Möglichkeit zur Modifizierung von Cyanin-farstoffen vom Typ des König'schen Salzes besteht in der Verbrückung der beiden Stickstoff-Zentren durch eine Kronenether-artige cyclische oder auch offenkettige Kronenethereinheit mit Donorendgruppen gemäß Formel XIII, ($m = 1$ bis 3, $A^{\ominus} =$ Anion)

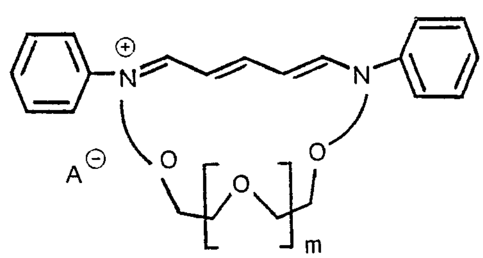

XIII

Auf diese Weise wird der Chromophor bei der Wirt-Gast-Wechselwirkung, d.h. beim Einnisten des Kations, durch die positive Ladung des Kations zusätzlich gestört. Das Kation wird durch die angeheftete Kronenetherstruktur unmittelbar an den empfindlichen Teil (Elektronenwolke) des Chromophors herangezogen bzw. fixiert und ist in der Lage, dieses chromophore System durch Anziehen von Elektronen aus der lockeren $\pi$-Elektronenwolke zu verändern, was die Farbeffekte hervorruft. Durch kürzere oder längere Kronenetherbrücken, die durch verschiedene Heteroatome bzw. durch den Einbau von starren aromatischen Strukturelementen wie Pyridinringen etc. modifiziert werden können, können diese Farbstoffe in gewünschter Weise bei der Komplexierung beeinflußt werden.

Eine andere Möglichkeit, Kronenether mit Cyaninstruktur vom Typ des König'schen Salzes herzustellen, besteht darin, von dem in para-Stellung mit einem Azakronenetherring versehenen Anilin auszugehen und bei der Komplexierung das einsame Elektronenpaar am para-Amin-Stickstoff des Kronenetherrings durch Einnisten des Ions empfindlich zu stören, so dß dieses einsame Elektronenpaar nur noch in geringem Maße zur Mesomerie zur Verfügung steht, was Farbänderungen erzwingt.

Die Erfindung umfaßt auch den Einsatz von Komplexliganden in Form eines cyclischen Petids oder eines Peptids, welches in Gegenwart des Ions oder der polaren Substanz die erforderliche Sekundär-, Tertiär- oder Quartärstruktur annimmt. So können auch Naturstoff-Ionophore wie Valinomycin, Nonactin Gramacidin und ähnliche Peptide für die erfindungsgemäßen ionenselektiven Farbreaktionen bzw. Farbstets eingesetzt werden. Hier geht man wie folgt vor: In Valinomycin wird ein Farbstoffsalz, z.B. 2,4-Dinitrophenylhydrazoniumchlorid, Natriumpikrat oder ein ähnlicher Farbstoff, eingelagert. Der Valinomycin-Farbstoff-Komplex hat im allgemeinen eine andere Farbe bzw. Absorptionsmaximum als der freie Farbstoff. Im zweiten Schritt wird zu diesem Valinomycin-Farbstoffkomplex die in ihrer Konzentration zu testende Salzlösung zugegeben, wobei das kaliumspezifische Valinomycin die in der Lösung vorhandenen Kalium-Ionen komplexiert, wodurch der Farbstoff aus dem Valinomycin-Hohlraum verdrängt wird und in Lösung nun wieder eine andere Farbe bzw. Absorptionsspektrum aufweist.

Auf diese Weise kann die Kaliumspezifität des Valinomycins für Farbtests ausgenutzt werden. Als einfaches Farbstoffsystem, das sich zur Einlagerung bzw. Anlagerung an Valinomycin und nachfolgende Verdrängung mit Kalium-Ionen eignet, können insbesondere Hydraziniumsalze mit verschiedenen angeknüpften mesomeren Systemen, wie z.B. Azo-Funktionen, eingesetzt werden. Es werden sterisch möglichst wenig anspruchsvolle Farbstoffkationen verwendet, die mit Valinomycin einen schwachen, aber doch hinreichend stabilen Komplex bilden, der auch nicht zu stark sein darf, so daß er danach mit Kalium-Ionen, auch in geringer Konzentration wieder quantitativ zerlegt werden kann.

Analoge Versuche mit Kronenethern, wie dem Kronenether [18] Krone-6, die also eine vergleichbare Struktur wie das Valinomycin und Nonactin haben, ließen deutliche Farbeffekte bzw. Änderungen der längerwelligen Absorption des Farbstoffs im an den Kronenether gebundenen Zustand und in freiem Zustand erkennen. Die Anwendung sowohl von kationischen Farbsalzen wie 2,4-Dinitrophenylhydrazonium-Ionen als auch anionischen Farbträgern wie Pikrationen bieten eine Fülle von Variations- und Anwendungsmöglichkeiten, deren Wahl von Fall zu Fall, d. h. auf die eingesetzten Komponenten exakt maßgeschneidert werden kann. Als weiterer Farbstoff ist bevorzugt die Azo-Verbindung XIV geeignet.

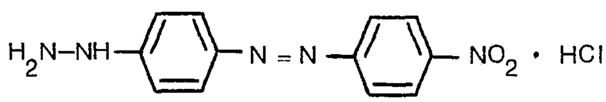

XIV

Von den neutralen Gastmolekülen, die erfindungsgemäß selektiv nachgewiesen werden können, sind insbesondere zu nennen Harnstoff, Thioharnstoff, Ammoniumsalze wie Cholin, Guanin, Guanidin, Harnsäure, Creatinin, Aminosäuren und Zucker, und zwar erfindungsgemäß aufgrund ihrer Farbeffekte, sowohl qualitativ als auch quantitativ. Zur quantitativen Bestimmung von Harnstoff aus wäßrigen Lösungen kann ein Chinolinligand synthetisiert werden, der sowohl für Harstoff als auch Thioharnstoff geeignet ist (siehe hierzu Tetrahedron Letters Nr. 3, 309—312, 1978).

Das erfindungsgemäße Verfahren unter Verwendung der neuartigen Farbstoffsysteme ist von großem Interesse, da es ohne weiteres eine Fülle von Messungen und Berechnungen erlaubt, die sich z.B. auf die Ionenselektivität, bedingt durch die Komplexkonstanten gegenüber verschiedenen Ionen und den Einfluß von Ionen bestimmter Ladungsdichte auf das chromophore System verschiedenster Farbstofftypen im Hinblick auf Extinktions- und Absorptionseigenschaften erstrecken. In vielen Fällen sind Vorausberechnungen mit Molekülorbital-Methoden möglich.

Eine weitere strukturelle Variante intramolekularer Ionophor-/Chromophor-Kombinationen der Typen XV und XVI zeigt eine andersartige Möglichkeit der Selektivitäts-Beeinflussung: Die im Kronenetherhohlraum fixierten Kationen werden zusätzlich von dem an einer kürzeren oder längeren Seitenkette befindlichen Donorzentrum koordiniert, die eine phenolisch oder $CH_2O^{\ominus}$-Gruppe oder $COO^{\ominus}$, $SO_3^{\ominus}$ sein kann. Hier sind zwei Einflüsse kombiniert: Die negative Ladung der Seitenkette bindet auf elektrostatischem Wege das Kation stärker, als dies durch einen Kronenetherrung aufgrund der Ion-Dipol-Wechselwirkungen möglich wäre. Das Kation muß aber dennoch in den Kronenetherring hineinpassen, d.h. es wird dort auf seine Größe hin geprüft und selektiert. Insgesamt beobachtet man experimentell höhere Komplexkonstanten aufgrund der stärkeren Bindung des Kations an den Liganden, eine höhere Selektivität des im Kronenetherring eigebetteten Kations sowie als Folge davon starke Beeinflussung des Chromophorsystems aufgrund der günstigen Lage des Gleichgewichts gegenüber dem bischer beschriebenen Liganden und eine besondere Selektivität gegenüber Kationen mit hoher Ladungsdichte, beispielsweise $Ca^{2+}$-Ionen. Solche Ligandsysteme können nach Optimierung auf bestimmte Kationen, möglicherweise zur spezifischen Unterscheidung von einfach/mehrfach geladenen Kationen herangezogen werden.

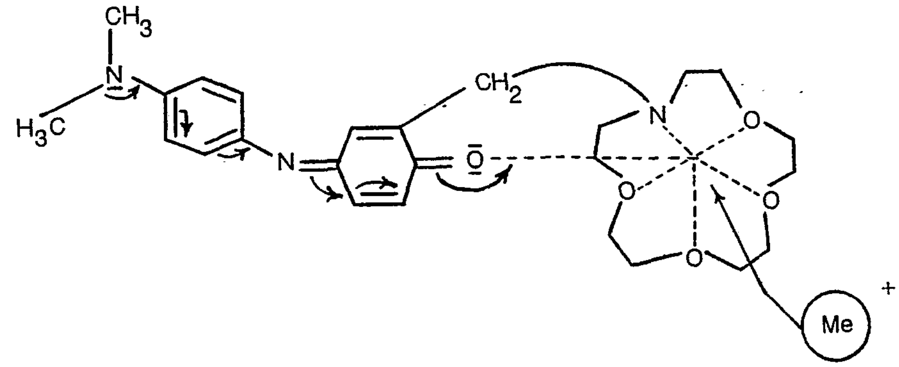

XV

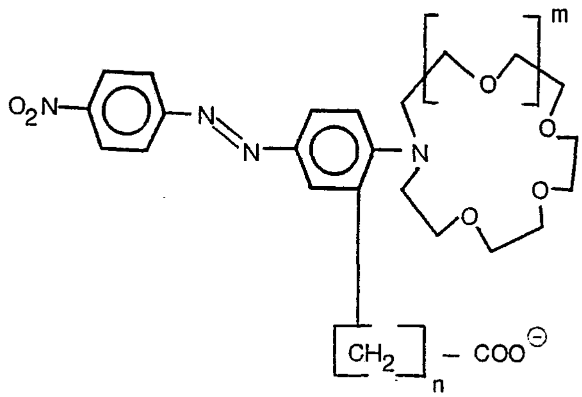

XVI

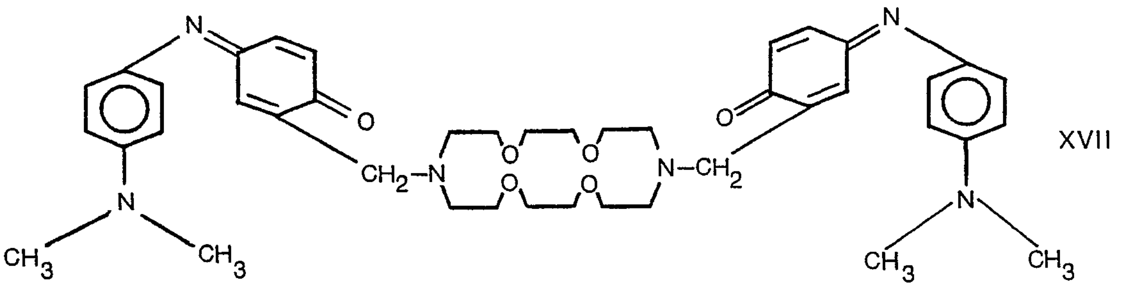

XVII

Das neue Verfahren ermöglicht eine Reihe von neuen praktischen Anwendungen:

a) Mit Hilfe der Kronenetherfarbstoffe lassen sich Indikatoren bzw. Sonden entwickeln, etwa zum Nachweis des Phasentransfers von Salzen, zum Studium des Ionentransportes durch lipophile Medien, z.B. künstlichen und biologischen Membranen, d.h. zu Untersuchungen im Zusammenhang mit membranologischen forschungen in physiologischer bzw. pathologischer Hinsicht.

b) Eine weitere Anwendung liegt in der Möglichkeit des ionen-selektiven Anfärbens von Gewebeschnitten. Eine gegebenenfalls quantitative Auswertung erlaubt hierbei Rückschlüsse auf Art und Konzentration von Ionen, welche sich in der Verschiebung von $\lambda_{max}$ und Extinktion bemerkbar machen.

c) Der Einsatz chromophorer Cryptand-Systeme gewährleistet vor allem im wäßrigen Medien neben wasentlich höherer Komplexstabilität eine verbesserte Selektivität. Neben dem qualitativen und quantitativen photometrischen Nachweis der Alkali-, Erdalkali- sowie Ammonium-Ionen mit Hilfe von Wirtschromophoren verschiedener Hohlraumgröße in Lösungen, wie z.B. Blut oder Seren und anderen Körperflüssigkeiten, lassen sich diese auch auf Trägermaterialien identifizieren. Hierbei ist die Verwendung als Sprühreagenz für Ionenchromatogramme oder Siliconabdrücken aus dem Bereich der medizinischen Diagnostik möglich. Nach dem Muster der Thermographie lassen sich entweder durch Besprühen von Gewebebereichen oder deren Abdrücken mit ionenselektiven Farb- und Fluoreszenzstoffen physiologische bzw. pathlogische Salzkonzentrationen durch Farbveränderungen sichtbar machen. Es ergibt sich dadurch die Möglichkeit, krankhafte Stellen wie etwa Krebsgewebe von gesundem Material zu unterscheiden.

d) Aus der Kenntnis über Komplexierung von organischen Ammonium- und Guanidiniumsalzen sowie von Neutralmolekülen (z.B. Harnstoff und CH-acide Verbindungen) ist erfindungsgemäß nunmehr der Nachweis mit geeigneten Wirtschromophoren ermöglicht. Der Nutzen in medizinischer und biochemischer Hinsicht liegt hierbei im spezifischen photometrischen Nachweis bestimmter körpereigener Substanzen und der kinetischen Verfolgung enzymatischer Prozesse. Im Hinblick darauf ist an die Anheftung lipophiler Hohlräume in Form von endolipophilen bzw. endohydrophoben makrocyclischen Verbindungen mit exohydrophiler oder exopolarophiler Molekularperipherie an empfindliche, d.h. chromophorbeeinflussende, Positionen eines der Farbstoffe gedacht, die die Einhüllung eines passenden Neutralteilchens erlauben und wahrscheinlich durch H-Brücken bedingt eine Absorptions bzw. Extinktionsveränderung hervorrufen.

Weitere experimentelle Darlegungen gehen aus den folgenden Beispielen hervor.

Beispiel 1

Photometrische Bestimmung von Kalium mit einem ionenselektiven Farbstoff, der den Chromophor über ein mesomeres System kovalent gebunden enthält. Der ionenselektive Farbstoff der Formel IV′ (n= 1) wird in Chloroform gelöst und mit einer Lösung, die Kalium-Ionen enthält, geschüttelt. Der mit Kalium komplexierte Farbstoff tritt in der wäßrige Phase über und kann bei 600 nm im Photometer quantitative, in Abhängigkeit von der Aktivität vorhandener Kalium-Ionen, bestimmt werden.

Der Farbstoff (5-Nitro-1,3-thiazol-2-azo-[3-(12-hyroxy-1,4,7,10-tetraoxadodecyl]-4-hydroxybenzol) wird erhalten durch Umsetzung von 2-Amino-5-nitrothiazol in 85 proz. Phosphorsäure mit Natriumnitrit und anschließender Zugabe von Benzo [15] krone-5. Die intensiv rote Suspension wird mit Wasser versetzt und mit Chloroform extrahiert. Nach dem Trocknen mit Natriumsulfat und Entfernen des Lösungsmittels im Vakuum wird auf Kieselgel säulenchromatographisch aufgetrennt. Mit Chloroform erhält man zunächst den ringgeschlossenen Farbstoff 5-Nitro-1,3-thiazol-2-azo[1,4,7,10,13-pentaoxa[13](3,4)-benzophan (Formel VII). Durch Zufügen von 5% Ethanol zum Eluens erhält man den ringoffenen Farbstoff der Formel 1 IV′, n=1. Beide Farbstoffe lassen sich durch Auflösen in Essigester und Zufügen von Petrolether 60—90°C kristallin erhalten. Schmelzpunkt 169—171°C (VII) und 112—115°C (IV′).

Beispiel 2

Zur Untersuchung der Eigenschaften des in Beispiel 1 genannten ionenselektiven Farbstoffs für Streifenteste wurde die Lösung des Farbstoffs auf einen Filterpapierstreifen getropft und dieser Streifen getrocknet. Der so behandelte Papierstreifen wird bei Zugabe von Probelösungen, die Kaliumionen enthalten, dunkelrot gefärbt. Neben dieser qualitativen Nachweismethode kann der Angefärbte Streifen in einem Reflexionsphotometer quantitativ ausgewertet werden.

Beispiel 3

Selektive photometrische Bestimmung von Kalium mit einem Liganden, der einen Chromophor kovalent gebunden trägt (Verbindung des Typs Formel IV, worin n = 1 und

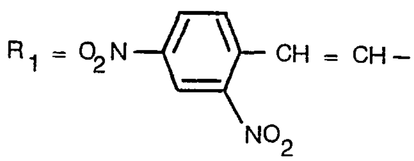

erhalten durch dreistündige Umsetzung von 2,4-Dinitrotoluol mit N-(p-Formylphenyl)aza[15]krone-5 mit einigen Tropfen Pipiridin bei 100°C. Die erstarrte Schmelze wird in wenig Essigester gelöst, filtriert und im Vakuum eingeengt. Es wird säulenchromatographisch an Kieselgel mit Essigester/5—10% Ethanol gereinigt. Beim Einengen des Eluats kristallisiert das 2,4-Dinitro-4′-(4,7,10,13-tetraoxa-1-azo-cyclopentadec-1-yl) stilben aus. Schmelzpunkt 143—146°C.

7,5 mg Farbstoff werden in 250 ml Methanol gelöst. Außerdem werden hergestellt 0,1 M Lösungen von je Calciumchlorid, Natriumchlorid, Kaliumchlorid, Lithiumchlorid und Magnesiumchlorid jeweils in 0.1 M Triäthanolamin-Hydrochlorid/NaOH-Puffer, pH = 7,0.

Außerdem wird eine Reihe von Lösungen mit 0,001 bis 0,1 M Kaliumchlorid hergestellt in jeweils 0,1 M Triäthanolamin-Hydrochlorid/NaOH-Puffer, pH = 7,0.

3 ml Farbstofflösungen werden mit 0,5 ml der jeweils zu untersuchenden Salzlösung versetzt bei 366 nm das Absorptionsverhalten im Photometer bestimmt. Dieser Farbstoff zeigt gegenüber Kalium ein anderes Spektrum als in Gegenwart von Lithium, Natrium, Calcium, Barium und Magnesium. Die Konzentration der Kalium-Ionen ist der Extinktion direkt proportional. Die Absorbtionsspektren einiger dieser Metallionen sind in Fig. 1 wiedergegeben.

Beispiel 4

Photometrische Bestimmung von Calcium und Lithium mit einem heterocyclischen Kronenether der Formel IV; wobei n=1 und

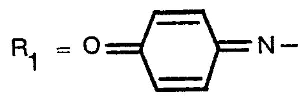

ist und somit der Chromophor kovalent verknüpft ist.

Das 1,4-Benzochinon-(4,7,10,13-tetraoxa-1-azacyclo-pentadec-1-yl)-phenylimin erhält man durch oxidative Kupplung von N-(4-Aminophenyl)aza[15]krone 5 mit Phenol, Dazu werden Silbernitrat mit Natriumchlorid und etwas Stärke mit einander umgesetzt, Natriumcarbonat und Phenol zugegeben und die Lösung von N-(4-Aminophenyl)aza[15]krone 5 in konzentrierter Salzsäure zugetropft. Nach der Umsetzung wird mit Essigester Ausgerührt, mit Natriumsulfat getrocknet und im Vakuum eingedampft. Der Rückstand kristallisiert aus Tetrahydrofuran mit Ether bei 0°C. Schmelzpunkt 55—55°C, $\lambda_{max}$ = 583 nm, log $\varepsilon$ = 4,77.

Der Farbstoff wird in Chloroform gelöst ($E_{578\ nm}$ = 0.8) und mit gleichen Volumenteilen wäßriger Salzlösung versetzt. Mit Lithium und Calcium wurde bei 578 nm eine Farbänderung gemessen, die der Konzentration der zu bestimmenden Ionen direkt proportional ist.

Durch oxidative Kupplung von $\alpha$-Naphthol mit N-(4-Aminophenyl)aza[15]krone 5 erhält man 1,4-Naphthochinon-4-(4,7,10,13-tetraoxa-1-azacyclopentadec-1-yl)-phenylimin vom Schmelzpunkt 124—125°C, $\lambda_{max}$ = 577 nm, log $\varepsilon$ = 4,41. Die Farbverschiebungen durch metallionen entsprechen denen des Benzochinonderivats.

Beispiel 5

Orangegefärbtes 2,4-Dinitrophenylhydrazinium-Hydrochlorid ($\lambda_{max}$ = 395 nm) wurde in Methanol/$H_2O$ gelöst und mit molaren Mengen Kronenether[18]Krone 6 versetzt. Man beobachtet eine Änderung der Absorption (Aufhellung) zwischen dem freien Farbstoff und dem im Ionophor-Komplex gebundenen ($\lambda_{max}$ = 384 nm). Man fügt hierzu eine Kaliumionen- oder Natriumionen-haltige wäßrige Methanol-Lösung, z.B. eine Natriumperchlorat- oder Kaliumrhodanid-Lösung, zu und beobachtet dann eine Farbvertiefung, da der freigesetzte Farbstoff wieder das anfängliche Absorptionsmaximum aufweist.

Ersetzt man den Kronenether in dem vorhergehenden Experiment durch andere Ionophore wie Valinomycin oder Nonactin, so beobachtet man ähnliche, je nach Ionophor mehr oder weniger stark ausgeprägte Farbeffekte bzw. Absorptionsänderungen.

Beispiel 6

In analoger Weise wie im Beispiel 3 beschrieben, wird 2,4-Dinitrotoluol mit N-(p-Formylphenyl)aza[18]krone 6 und einigen Tropfen Pipiridin 4—5 Stunden lang auf 100—110°C erhitzt. Man löst in Dichlormethan und chromatographiert an Kieselgel mit Essigester/Chloroform 1:1. Nach dem Umkristallisieren aus Essigester/Ethanol 1:1 erhält man den Farbstoff 2,4-Dinitro-4'-(4,7,10,13,16-pentaoxa-1-aza-cyclooctadec-1-yl)- stilben vom Schmelzpunkt 90—91°C. Die Absorptionsspektren des Farbstoffs und seiner Komplexe mit Barium-, Ammonium-, Calcium-, Kalium-, Natrium-, Rubidium- und Lithium-ioden sind in der Fig. 2 wiedergegeben.

Beispiel 7

In analoger Weise wie im Beispiel 4 beschrieben, erhält man durch oxidative Kupplung von N-(2-Hydroxy-benzyl)monoaza [15] krone-5 mit 4-Amino-N,N-dimethylanilin einen blauen Farbstoff, der sich mit Essigester extrahieren läßt. Nach dem Trocknen mit Magnesiumsulfat und Eintrocknen im Vakuum chromatographiert man an Kieselgel mit Ethanol/Essigester 1:9. Die Absorptionsspektren des Farbstoffs und seiner Komplexe mit Natrium-, Kalium-, Rubidium, Magnesium-, Calcium-, Barium-, Lithium- und Nickelionen sind in der Fig. 3 wiedergegeben.

Beispiel 8

In analoger Weise wie im Beispiel 7 beschrieben, erhält man aus N-(2-Hydroxybenzyl)-monoaza[18]krone 6 einen Farbstoff, dessen Spektrum im freien und komplexierten Zustand in Fig. 4 wiedergegeben ist.

Beispiel 9

Die Spektren von 1,8-Bis[1-(4-dimethylaminophenylimino)-p-benzochin-3-oxy]-3,6-dioxaoctan sind in freier Form und in komplexierter Form mit Kalium-, Natrium-, Lithium-, Rubidium-, Barium- und Calciumionen in der Fig. 5 wiedergegeben, Die Substanz wird hergestellt durch Reduktion von p-Nitroso-N-N-dimethylanilin mit Salzsäure und Zinkstaub und Umsetzung mit 1,8-Bis-(2-hydroxyphenoxy)-3,8-dioxaoctan in Gegenwart von Natriumhydroxid und Kaliumdichromat. Beim Ansäuern mit Eisessig fällt der blaue Farbstoff aus. Nach dem Austrocknen mit Aceton wird abgekühlt,

abgesaugt, mit wenig Aceton gewaschen. Man extrahiert mit Aceton im Heißextraktor und chromatographiert mit Essigester an Kieselgel. Den Farbstoff erhält man durch Zugabe von 5% Ethanol zum Essigester. Nach der Kristallisation aus Aceton/n-Heptan ist der Schmelzpunkt 112—115°C $\lambda_{max} = 572$ nm log $\varepsilon = 4{,}73$.

Beispiel 10

Das Spektrum des Farbstoffs IV, bei dem

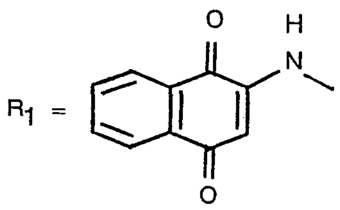

und n = 1 ist, nebst den Spektren der Metallionenkomplexe mit Calcium, Barium, Natrium, Lithium und Kalium ist in der Figur 6 wiedergegeben. Der Farbstoff wird gewonnen, indem man N-(4-Aminophenyl)aza[15]krone-5 und 1,4 Naphthochinon mit Kupferacetatmonohydrat in Ethanol suspendiert, 1 Stunde zum Sieden erhitzt und Luft hindurchleitet. Nach Entfernen des Lösungsmittels im Vakuum wird mit Wasser versetzt, mit Dichlormethan extrahiert, über Magnesiumsulfat getrocknet und im Vakuum einengt. Der ölige Rückstand wird mit Dichlormethan as Kieselgel gereinigt. Das Eluat enthält an fangs überschüssiges Naphthochinon und nach Zufügen von 5% Ethanol den gewünschten Farbstoff, der aus Essigester umkristallisiert werden kann.

**Patentansprüche**

1. Verfahren Bestimmung von Ionen oder polaren und/oder lipophilen Substanzen in Flüssigkeiten unter Verwendung von gegenüber dem zu bestimmenden Ion oder der polaren und/oder lipophilen Substanz selektiven Komplexiganden bzw. selektiven Wirtsmolekülen, dadurch gekennzeichnet, daß man das zu bestimmende Ion oder die polare und/oder lipophile Substanz einwirken laßt auf einen selektiven Komplexliganden bzw. ein selektives Wirtsmolekül aus der Gruppe der Cyclodextrane, Cyclophane, Kronenäther, Kryptanden, Podanden, Oligoester, Polyester, Oligoamide, Oligopeptide, Polypeptide, Oligoether und Polyether, welches durch eine direkte covalente Bindung mit einem Chromophor verknüpft ist und danach die Wellenlängenverschiebung im Absorptions- oder Emissionsspektrum oder die Extinktionsänderung mißt.

2. Verfahren gemäß Anspruch 1, dadurch gekennzeichnet, daß der covalent gebundene Chromophor ein Farbstoff mit Polyen-Struktur ist.

3. Verfahren gemäß Anspruch 1, dadurch gekennzeichnet, daß der covalent gebundene Chromophor ein Farbstoff mit Azo-Struktur ist.

4. Verfahren gemäß Anspruch 1, dadurch gekennzeichnet, daß der covalent gebundene Chromophor ein Farbstoff mit Merichinoid-, Chinon-, Anthrachinon-, Naphthochinon- oder Alizarin-Struktur ist.

5. Verfahren gemäß Anspruch 1, dadurch gekennzeichnet, daß der covalent gebundene Chromophor ein Farbstoff mit Polymethin-, Azomethin-, Cyanin- oder Merocyanin-Struktur ist.

6. Verfahren gemäß Anspruch 1, dadurch gekennzeichnet, daß der covalent gebundene Chromophor ein Farbstoff mit Pyrrol-, Indigo- oder Indophenol-Struktur ist.

7. Verfahren gemäß Anspruch 1, dadurch gekennzeichnet, daß der covalent gebundene Chromophor ein Farbstoff mit Phthalein- oder Stilben-Struktur ist.

8. Verfahren gemäß Anspruch 1 bis 7, dadurch gekennzeichnet, daß der Chromophor ein saurer Farbstoff oder ein Salz davon ist.

9. Verfahren nach einem der Ansprüche 1 bis 8, dadurch gekennzeichnet, daß das Salz des Farbstoffes ein Lithium-, Kalium-, Ammonium, Calcium-, Alkylammonium- oder Magnesium-, salz ist.

10. Verfahren nach einem der Ansprüche 1 bis 9, dadurch gekennzeichnet, daß der Farbstoff eine Carboxylat-, Sulfonat-, Phenolat- oder Thiophenolatgruppierung enthält.

11. Verfahren nach einem der Ansprüche 1 bis 10, dadurch gekennzeichnet, daß das zu bestimmende Ion ein Alkali-, Erdalkalioder Ammoniumion ist.

12. Verfahren nach einem der Ansprüche 1 bis 10,, dadurch gekennzeichnet, daß das zu bestimmende Ion das Eisen-, Zink-, Kupfer-, Kobalt-, Nickel-, Molybdän- oder Chromion ist.

13. Verfahren gemäß einem der Ansprüche 1 bis 9, dadurch gekennzeichnet, daß die nachzuweisende Substanz ein neutrales, polares, wasserlösliches Gastteilchen wie Harnstoff, Thioharnstoff, Guanidin, Harnsäure, Cholin, Creatin oder eine Aminosäure ist.

14. Verfahren nach einem der Ansprüche 1 bis 13, dadurch gekennzeichnet, daß das nachweissystem auf einem festen Träger wie Papier, Kunststoffolie, Glas, Aluminiumoxid, Siliciumoxid, Natur- oder Kunstfaser, oder Metall aufgebracht oder darin eingearbeitet ist.

15. Reagenz zur Bestimmung von Ionen oder polaren und/oder lipophilen Substanzen in Flüssigkeiten, enthaltend gegenüber dem zu bestimmenden Ion oder der polaren und/oder lipophilen Substanz selektiven Komplexliganden bzw. selektiven Wirtsmolekülen, dadurch gekennzeichnet, daß die selektiven Komplexliganden bzw. selektive Wirtsmoleküle Substanzen aus der Gruppe der Cyclodextrane, Cyclophane, Kronenäther, Kryptanden, Podanden, Oligoester, Polyester, Oligoamide, Oligopeptide, Polypeptide, Oligoether und Polyether sind, welche durch eine direkte covalente Bindung mit einem Chromophor verknüpft sind, wobei als Komplexliganden makrocyclische Imine mit insgesamt 14 bis 60 Ringatomen ausgenommen sind.

## Claims

1. Process for the determination of ions or of polar and/or lipophilic substances in liquids with the use of complex ligands or selective host molecules which are selective towards the ion or the polar and/or lipophilic substance to be determined, characterised in that one allows the ion or the polar and/or lipophilic substance to be determined to act upon a selective complex ligand or a selective host molecule from the group of cycloextrans, cyclophanes, crown ethers, cryptands, podands, oligoesters, polyesters, oligoamides, oligopeptides, polypeptides, oligoethers and polyethers which is linked by a direct covalent bond with a chromophore and thereafter measures the wavelength displacement in the absorption or emission spectrum or the extinction change.

2. Process according to claim 1, characterised in that the covalently-bound chromophore is a dyestuff with polyene structure.

3. Process according to claim 1, characterised in that the covalently-bound chromophore is a dyestuff with azo structure.

4. Process according to claim 1, characterised in that the covalently-bound chromophore is a dyestuff with meriquinoid, quinone, anthraquinone, naphthoquinone or alizarine structure.

5. Process according to claim 1, characterised in that the covalently-bound chromophore is a dyestuff with polymethine, azomethine, cyanine or merocyanine structure.

6. Process according to claim 1, characterised in that the covalently-bound chromophore is a dyestuff with pyrrole, indigo or indophenol structure.

7. Process according to claim 1, characterised in that the covalently-bound chromophore is a dyestuff with phthalein or stilbene structure.

8. Process according to claims 1 to 7, chracterised in that the chromophore is an acidic dyestuff or a salt thereof.

9. Process according to one of claims 1 to 8, chracterised in that the salt of the dyestuff is a lithium, potassium, ammonium, calcium, alkylammonium or mangnesium salt.

10. Process according to one of claims 1 to 9, chracterised in that the dyestuff contains a carboxylate, sulphonate, phenolate or thiophenolate grouping.

11. Process according to one of claims 1 to 10, characterised in that the ion to be determined is an alkali metal, alkaline earth metal or ammonium ion.

12. Process according to one of claims 1 to 10, characterised in that the ion to be determined is the iron, zinc, copper, cobalt, nickel, molybdenum or chromium ion.

13. Process according to one of claims 1 to 9, characterised in that the substance to be detected is a neutral, polar, water-soluble guest particle, such as urea, thiourea, guanidine, uric acid, chlorine, creatine or an amino acid.

14. Process according to one of claims 1 to 13, characterised in that the detection system is applied to or incorporated in a solid carrier, such as paper, synthetic resin foil, glass, aluminium oxide, silicon oxide, natural or synthetic fibre or metal.

15. Reagent for the determination of ions or of polar and/or lipophilic substances in liquids, containing complex ligands or host molecules selective towards the ion or the polar and/or lipophilic substance to be determined, characterised in that the selective complex ligands or selective host molecules are substances from the group of the cyclodextrans, cyclophanes, crown ethers, cryptands, podands, oligoesters, polyesters, oligoamides, oligopeptides, polypeptides, oligoethers and polyethers, which are connected by a direct covalent bond with a chromophore, whereby, as complex ligands, macrocyclic imines with a total of 14 to 60 ring atoms are excluded.

—

## Revendications

1. Procédé de dosage d'ions ou de substances polaires et/ou lipophiles dans des liquides en utilisant des ligands sélectifs ou des molécules réceptrices sélectives à l'égard de l'ion ou de la substance polaire et/ou lipophile à doser, caractérisé en ce qu'on fait agir l'ion ou la substance polaire et/ou lipophile sur un ligand sélectif ou sur une molécule réceptrice sélective, choisi dans le groupe constitué par les cyclodextranes, les cyclophanes, les éthers à couronne, les cryptands, les podands, les oligo-esters, les polyesters, les oligo-amides, les oligopeptides, les polypeptides, les oligo-éthers et les poly-éthers, lié à un chromophore par une liaison covalente directe, après quoi on mesure le décalage des longueurs d'onde dans le spectre d'absorption ou d'émission, ou la variation de l'extinction.

2. Procédé selon la revendication 1, caractérisé en ce que le chromophore lié par covalence est un colorant ayant une structure de polyène.

3. Procédé selon la revendication 1, caractérisé en ce que le chromophore lié par covalence est un colorant ayant une structure azoïque.

4. Procédé selon la revendication 1, caractérisé en ce que le chromophore lié par covalence est un colorant ayant une structure de mériquinoléine, de quinone, d'anthraquinone, de naphtoquinone ou d'alizarine.

5. Procédé selon la revendication 1, caractérisé en ce que le chromophore lié par covalence est un colorant ayant une structure de polyméthine, d'azométhine, de cyanine ou de mérocyanine.

6. Procédé selon la revendication 1, caractérisé en ce que le chromophore lié par covalence est un colorant ayant une structure de pyrrol, d'indigo ou d'indophénol.

7. Procédé selon la revendication 1, caractérisé en ce que le chromophore lié par covalence est un colorant ayant une structure de phtaléine ou de stilbène.

8. Procédé selon l'une quelconque des revendications 1 à 7, caractérisé en ce que le chromophore est un colorant acide ou un sel de celui-ci.

9. Procédé selon l'une quelconque des revendications 1 à 8, caractérisé en ce que le sel du colorant est un sel de lithium, de potassium, d'ammonium, de calcium, d'alkylammonium ou de magnésium.

10. Procédé selon l'une quelconque des revendications 1 à 9, caractérisé en ce que le colorant renferme un groupe carboxylate, sulfonate, phénolate ou thiophénolate.

11. Procédé selon l'une quelconque des revendications 1 à 10, caractérisé en ce que l'ion à doser est un ion alcalin, alcalino-terreux ou d'ammonium.

12. Procédé selon l'une quelconque des revendications 1 à 10, caractérisé en ce que l'ion à doser est l'ion de fer, de zinc, de cuivre, de cobalt, de nickel, de molybdène ou de chrome.

13. Procédé selon l'une quelconque des revendications 1 à 9, caractérisé en ce que la substance à doser est une particule de passage neutre, polaire, hydrosoluble, comme l'urée, la thio-urée, la guanidine, l'acide urique, la choline, la créatine ou un amino-acide.

14. Procédé selon l'une quelconque des revendications 1 à 13, caractérisé en ce que le système de mise en évidence est appliqué sur, ou incorporé dans, un support solide comme le papier, une matière synthétique en feuille, le verre, l'oxyde d'aluminium, l'oxyde de silicium, une fibre naturelle ou synthétique ou un métal.

15. Réactif pour le dosage d'ions de substances polaires et/ou lipophiles dans des liquides contenant des ligands sélectifs ou des molécules réceptrices sélectives à l'égard de l'ion ou de la substance polaire et/ou lipophile à doser, caractérisé en ce que les ligands sélectifs ou les molécules réceptrices sélectives sont des substances choisies dans le groupe des cyclodextranes, de cyclophanes, des éthers à couronne, des cryptates, des podands, des oligo-esters, des polyesters, des oligo-amides, des oligopeptides, des polypeptides, des oligo-éthers et des polyéthers, qui sont liées par une liaison covalente directe à un chromophore, les imines macrocycliques ayant au total entre 14 et 60 atomes dans le cycle étant exclues en tant que ligands.

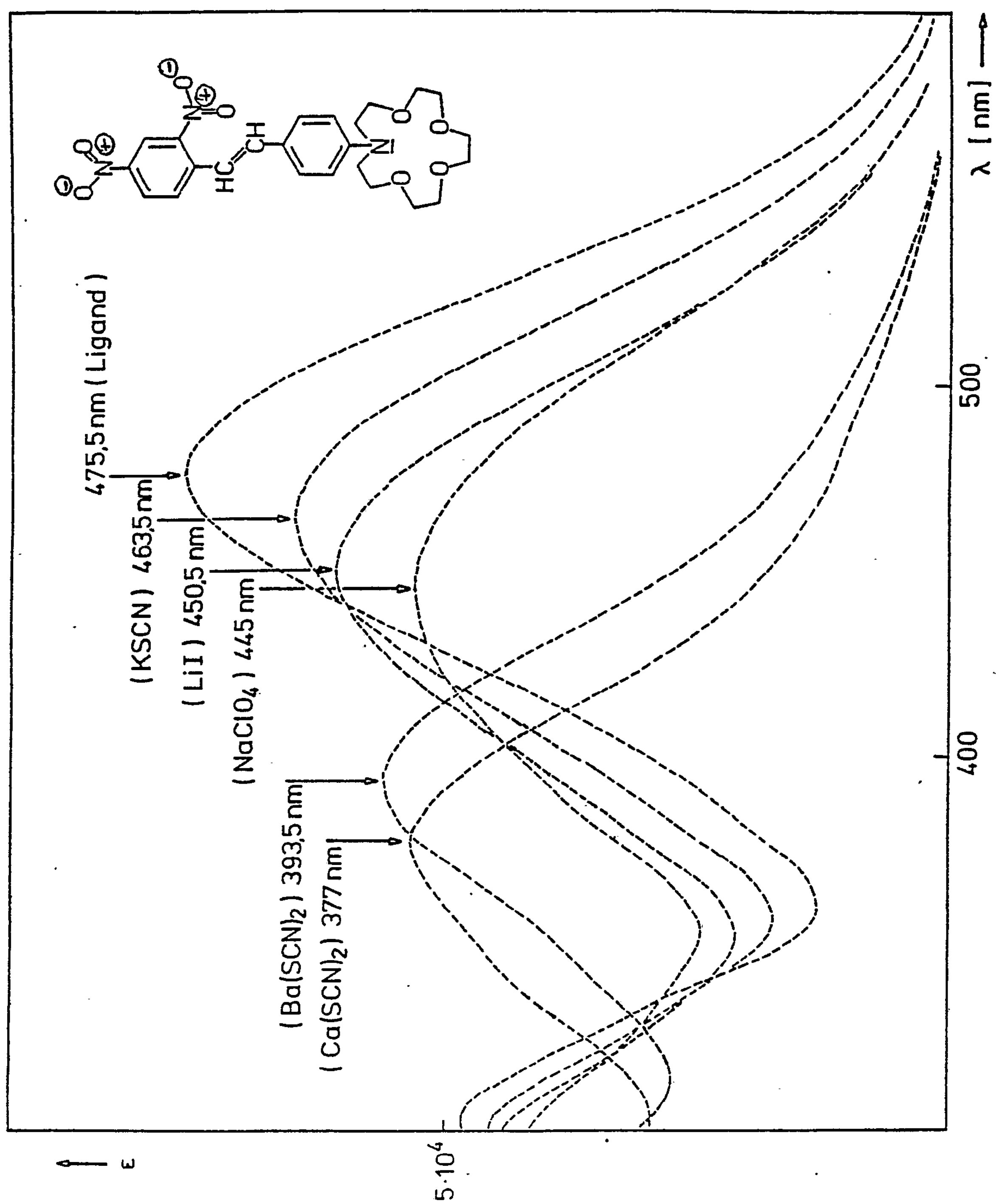
475,5 nm (Ligand)
(KSCN) 463,5 nm
(LiI) 450,5 nm
(NaClO4) 445 nm
(Ba(SCN)2) 393,5 nm
(Ca(SCN)2) 377 nm
500
400
λ [nm]
5·10^4
ε

Figur 1

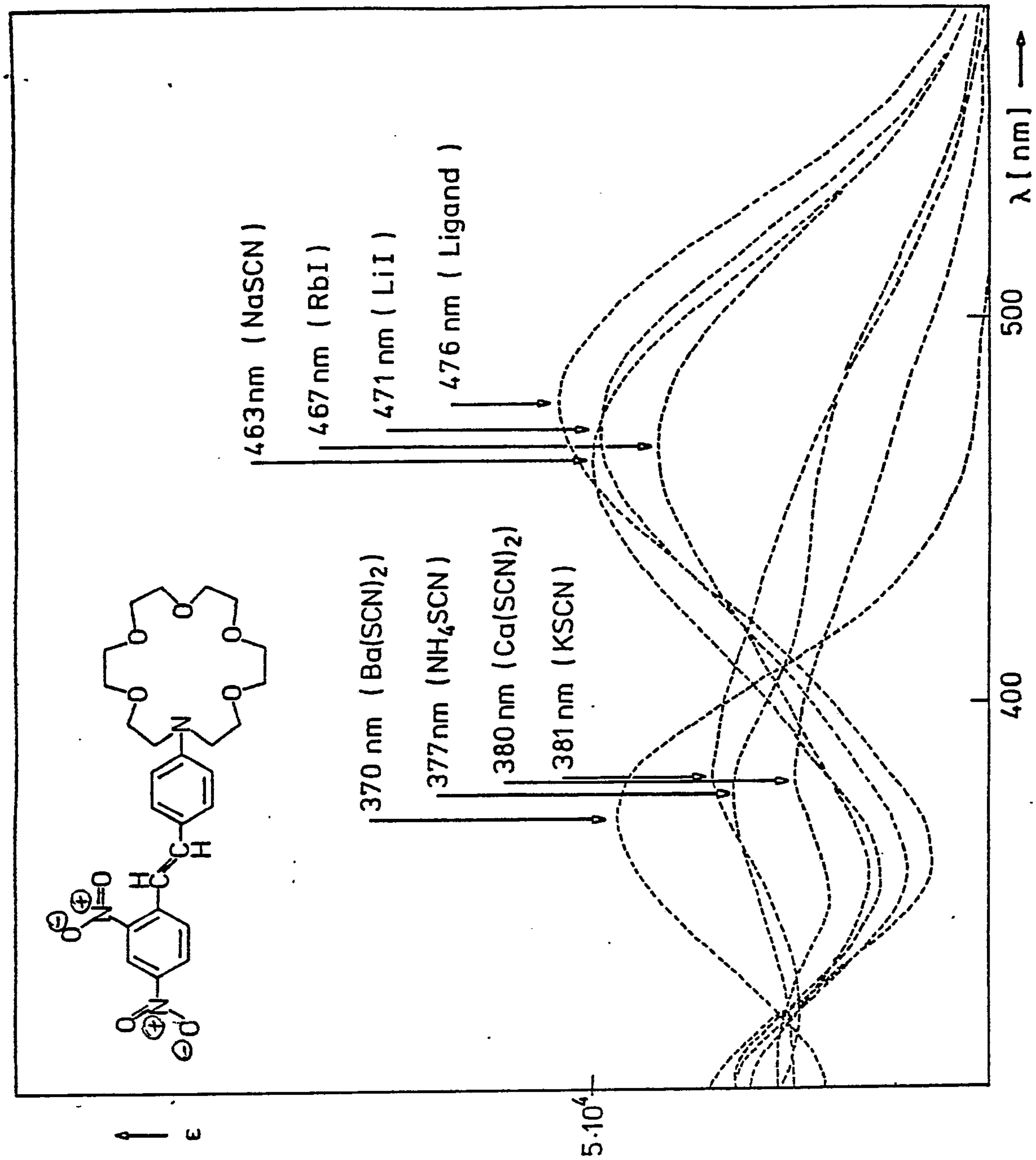
463nm ( NaSCN )
467 nm ( RbI )
471 nm ( LiI )
476 nm ( Ligand )
370 nm ( Ba(SCN)2 )
377nm ( NH4SCN )
380 nm ( Ca(SCN)2 )
381 nm ( KSCN )
400
500
λ [ nm ]
5·10⁴
ε

Figur 2

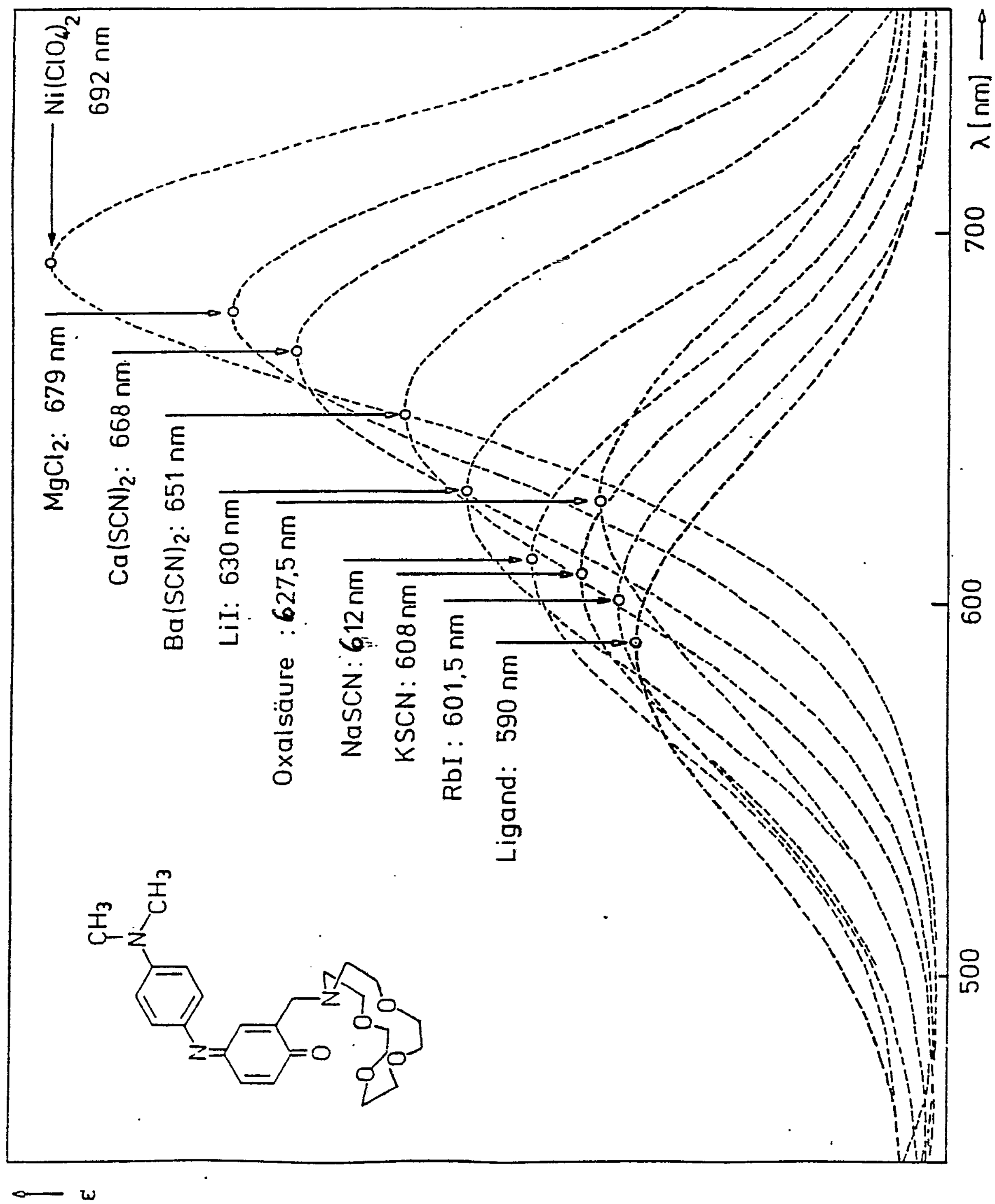
Ni(ClO4)2
692 nm
MgCl2: 679 nm
Ca(SCN)2: 668 nm
Ba(SCN)2: 651 nm
LiI: 630 nm
Oxalsäure : 627,5 nm
NaSCN: 612 nm
KSCN: 608 nm
RbI: 601,5 nm
Ligand: 590 nm
700
600
500
λ [nm]
ε

Figur 3

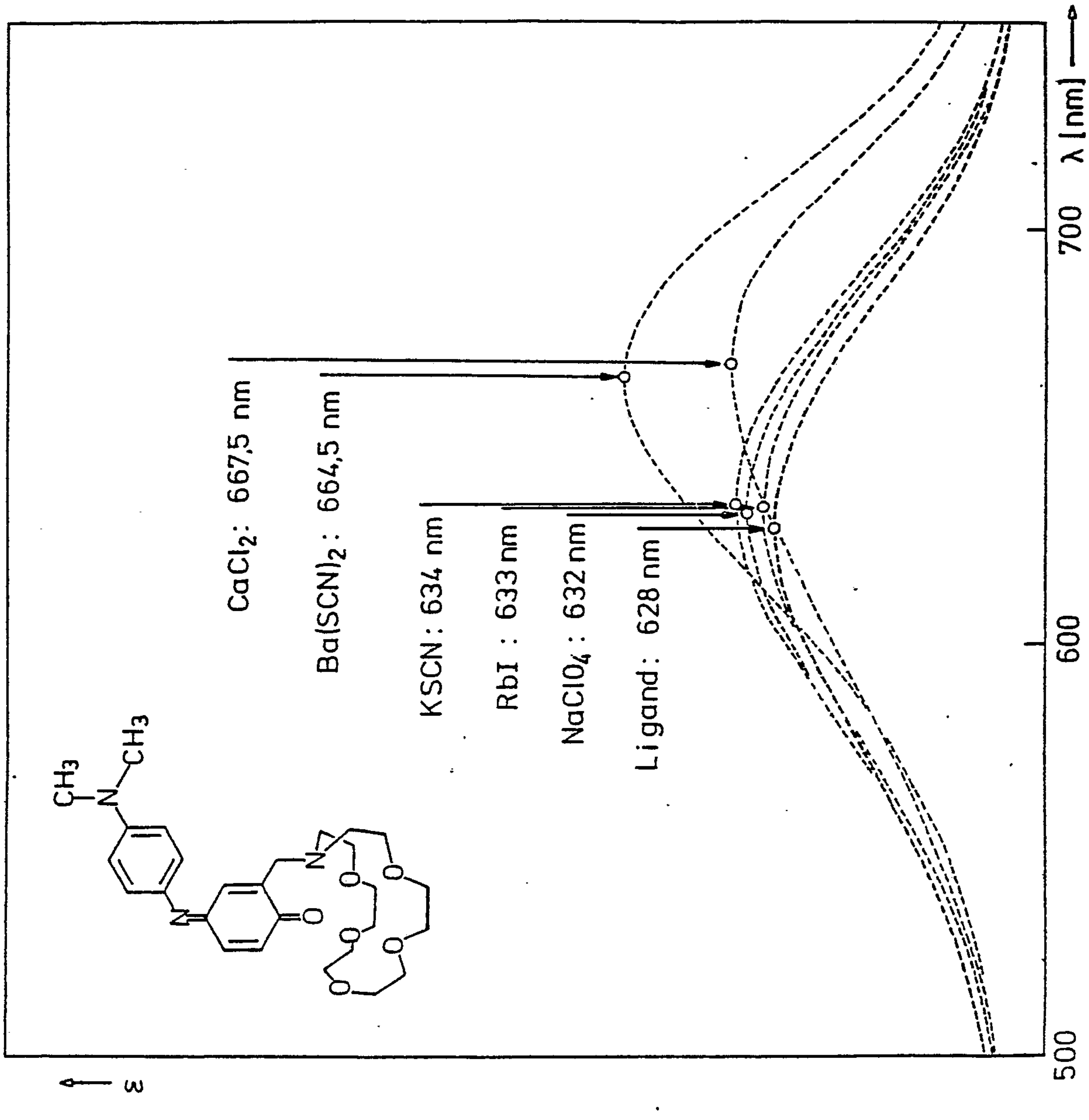
CaCl$_2$: 667,5 nm
Ba(SCN)$_2$: 664,5 nm
KSCN: 634 nm
RbI: 633 nm
NaClO$_4$: 632 nm
Ligand: 628 nm
500
600
700
λ [nm]
ε

Figur 4

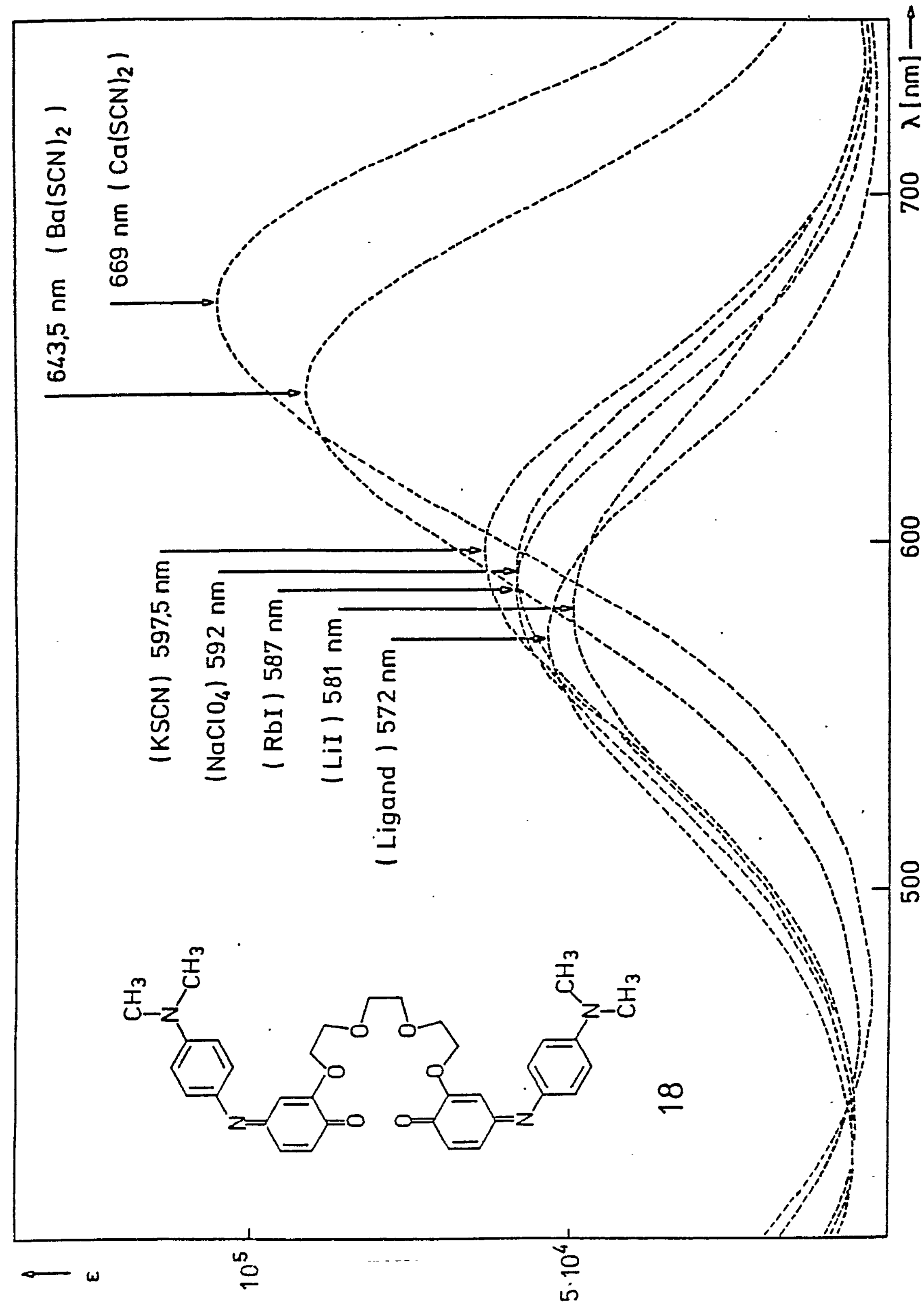
643,5 nm ( Ba(SCN)2 )
669 nm ( Ca(SCN)2 )
(KSCN) 597,5 nm
(NaClO4) 592 nm
( RbI ) 587 nm
( LiI ) 581 nm
( Ligand ) 572 nm
18
500
600
700
λ [nm]
ε
10^5
5·10^4

Figur 5

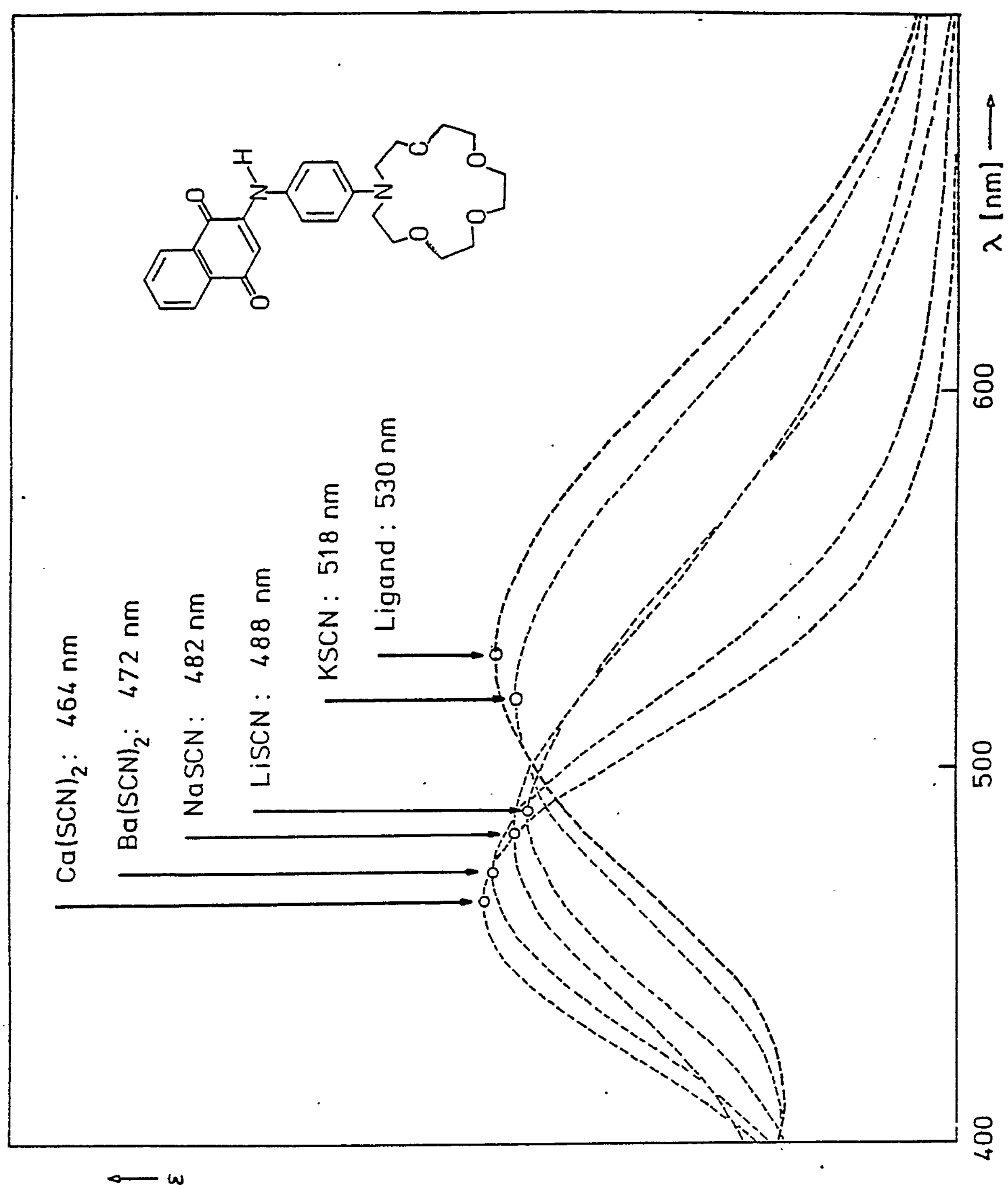
Ca(SCN)2 : 464 nm
Ba(SCN)2 : 472 nm
NaSCN : 482 nm
LiSCN : 488 nm
KSCN : 518 nm
Ligand : 530 nm
400
500
600
λ [nm]
ε

Figur 6